(19) Europäisches Patentamt
European Patent Office
Office européen des brevets

(11) Publication number: 0 216 424 A1

(12) **EUROPEAN PATENT APPLICATION**

(21) Application number: 86201606.0

(22) Date of filing: 17.09.86

(51) Int. Cl.[4]: C07D 233/64 , A01N 43/50

(30) Priority: 20.09.85 GB 8523255

(43) Date of publication of application: 01.04.87 Bulletin 87/14

(84) Designated Contracting States: AT BE CH DE FR GB IT LI LU NL SE

(71) Applicant: SHELL INTERNATIONALE RESEARCH MAATSCHAPPIJ B.V.
Carel van Bylandtlaan 30
NL-2596 HR Den Haag(NL)

(72) Inventor: Pettman, Roger Bruce
Broomhill House
Wychling Nr. Doddington(GB)

(74) Representative: Hunter, Keith Roger Ian et al
4 York Road
London SE1 7NA(GB)

(54) **Imidazoles, their preparation and their use as fungicides.**

(57) The invention provides imidazoles of general formula

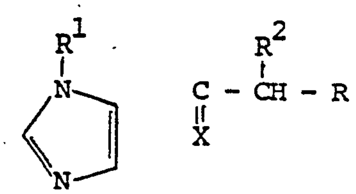

(I)

and acid-addition salts thereof, wherein R represents an optionally substituted phenyl group, $R^1$ represents an optionally substituted alkyl, cycloalkyl, alkenyl, aryl or aralkyl group, $R^2$ represents a hydrogen atom, a cyano group, a $-CONH_2$ group, or an optionally substituted alkyl group, and X represents an oxygen atom or a $=NOR^3$ moiety where $R^3$ is a hydrogen atom or an optionally substituted alkyl, cycloalkyl, alkenyl, alkylcarbonyl, aryl or aralkyl group; processes for their preparation, and their use as fungicides.

EP 0 216 424 A1

## IMIDAZOLES, THEIR PREPARATION AND THEIR USE AS FUNGICIDES

This invention relates to imidazoles, to a process for their preparation, and to their use as fungicides.

Belgian Patent No. 852,897 (Bayer) discloses a class of compounds of formula

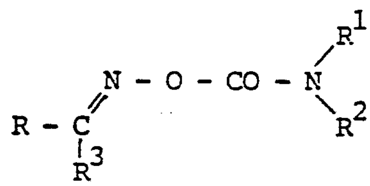

as insecticides, acarides and nematicides, wherein R designates an optionally substituted alkyl, cycloalkyl, aryl or aralkyl group, $R^1$ is a hydrogen atom or an alkyl group, $R^2$ is a hydrogen atom or an alkyl, alkenyl, alkynyl, haloalkyl, haloalkenyl or alkoxyalkyl group, $R^3$ is an optionally substituted nitrogen-containing heterocyclic group or a group -$CR^4R^5R^6$, where $R^4$ and $R^5$, which can be the same or different, represent hydrogen, an alkyl group or together form a pentamethylene or tetramethylene group and $R^6$ designates an optionally substituted nitrogen-containing heterocyclic group. The above compounds may be prepared from corresponding oximes of formula

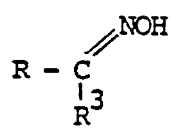

as intermediates. All nitrogen containing heterocyclic groups mentioned and exemplified for $R^3$ and $R^6$ are joined to the remainder of the molecule via the nitrogen atom, i.e. optionally substituted pyrazolyl-(1), imidazolyl-(1), 1,2,4-triazolyl-(1), 1,2,3-triazolyl-(1), 1,3,4-triazolyl-(1), indazolyl-(1), benzimidazolyl-(1), and benzotriazolyl-(1).

GB-A-2092 569 (Farmos) discloses a class of imidazoles of formula

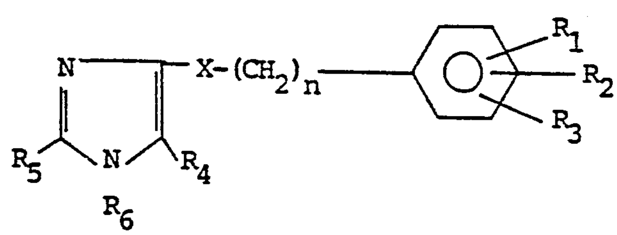

or

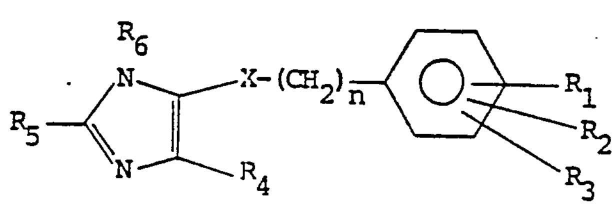

wherein each of $R_1$, $R_2$ and $R_3$, which can be the same or different, is hydrogen, chloro, bromo, fluoro, methyl, ethyl, methoxy, amino, hydroxy or nitro; $R_4$ is hydrogen or an alkyl radical of 1 to 7 carbon atoms; $R_5$ is hydrogen or a straight or branched alkyl group of 1 to 5 carbon atoms or a

phenyl group; and $R_6$ is hydrogen or an alkyl radical of 1 to 7 carbon atoms or a substituted or unsubstituted benzyl, X is $CH_2$, -CHOH-or -CH=CH-; and n is an integer from 0 to 4 provided that $R_5$ and $R_5$ are simultaneously hydrogen only when n is 4 and X is -CH=CH-. It will be appreciated that when $R_6$ is hydrogen the two aforesaid formulae are effectively the same. When $R_6$ is substituted benzyl it preferably carried up to 3 substituents selected from the same radicals as $R_1$, $R_2$ and $R_3$. These imidazoles are disclosed as having pharmacological properties, including antithrombotic, antihypertensive, beta-blocking, antibacterial and antifungal activity. Compounds of the above formulae wherein X is -CHOH-are disclosed as being preparable by a Grignard reaction between the appropriate imidazole aldehyde and arylalkyl magnesium halide. Such a reaction is stated surprisingly to produce by-products corresponding to the above formulae but wherein X is -CO-. It is stated that these compounds wherein X is -CO-can be reduced, e.g. using sodium borohydride in ethanol, to be compounds wherein X is -CHOH-. The only compound of the above formulae wherein X is -CHOH-and n is 1 which is specifically named is 4-[2-(2',6'-dimethyl-phenyl)-1-hydroxyethyl]-2-methylimidazole (Example 3), and no reference is made to the corresponding compound where X is -CO-having resulted as by-product in the Grignard reaction employed in its preparation.

There has now been discovered a novel class of 5-substituted imidazole derivatives having useful fungicidal activity.

According to the present invention there are provided imidazoles of general formula

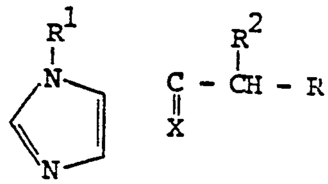

(I)

or an acid-addition salt thereof, wherein R represents an optionally substituted phenyl group, $R^1$ represents an optionally substituted alkyl, cycloalkyl, alkenyl, aryl or aralkyl group, $R^2$ represents a hydrogen atom, a cyano group, a $-CONH_2$ group, or an optionally substituted alkyl group, and X represents an oxygen atom or a $=NOR^3$ moiety where $R^3$ is a hydrogen atom, or an optionally substituted alkyl, cycloalkyl, alkenyl, alkylcarbonyl, aryl or aralkyl group.

Optional substituents include for example halogen atoms and alkyl, alkoxy, haloalkyl, haloalkoxy, hydroxy, cyano, nitro, amino, carboxy, alkoxycarbonyl, phenyl, phenoxy, phenylthio, alkylthio and alkylsulphonyl groups, any alkyl moiety present preferably having up to 4 carbon atoms.

R is preferably a phenyl group optionally substituted by 1 to 5 halogen atoms. Advantageously R is a phenyl group substitued by 1 to 3 halogen, preferably chlorine, atoms.

$R^1$ is preferably a $C_{1-10}$ alkyl, conveniently a $C_{1-6}$ alkyl, group, a $C_{3-8}$ cycloalkyl group, a $C_{2-6}$ alkenyl group, a phenyl group or benzyl group.

$R^2$ is preferably a $C_{1-6}$ alkyl group, or, more preferably, a hydrogen atom.

X preferably represents a $=NOR^3$ moiety wherein $R^3$ is a hydrogen atom, a $C_{1-6}$ alkyl group, a $C_{3-8}$ cycloalkyl group, a $C_{2-6}$ alkenyl group, a ($C_{1-6}$ alkyl) carbonyl group, or a benzyl group optionally substituted by one to three substituents selected from halogen atoms (such as chlorine, bromine or fluorine atoms) and methyl, methoxy, cyano and trifluoromethyl groups. $R^3$ is preferably a hydrogen atom, a $C_{1-6}$ alkyl group or a $C_{3-6}$ alkenyl group wherein the double bond is not adjacent the oxygen atom of the $=NOR^3$ moiety.

The present invention also provides a process for the preparation of an imidazole of formula I which comprises reacting an imidazole ester of formula

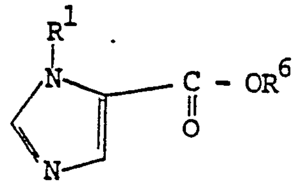

(II)

where $R^1$ is as defined above and $R^6$ is an alkyl group with a compound of formula

$R^7$ -$CH_2$ -R

wherein R is as defined above and $R^7$ is -CN or -$COOR^8$ where $R^8$ is an alkyl group, in the presence of a base, followed, optionally when $R^7$ is CN, by treatment with a strong acid to produce a compound of formula I wherein X is oxygen and $R^2$ is hydrogen or -$CONH_2$, optionally followed by reaction with an optionally substituted alkyl halide in the presence of a base to produce a compound of formula I wherein X is oxygen and $R^2$ is an optionally substituted alkyl group, optionally followed by reaction with a compound of formula $NH_2OR^{3'}$, where $R^{3'}$ is a hydrogen atom or an optionally substituted alkyl, cycloalkyl, alkenyl, aryl or aralkyl group, and optionally followed, in the case where $R^{3'}$ is hydrogen, by reaction with an acid derivative $R^6COL$, where $R^6$ is alkyl, preferably $C_{1-6}$ alkyl, and L is a leaving group, or with a compound of formula $R^9L'$ where $R^9$ is an optionally substituted alkyl, cycloalkyl, alkenyl or aralkyl group and L' is a leaving group.

The leaving group L may conveniently be a chlorine or bromine atom or a group -$OCOR^6$, where $R^6$ is as defined above.

The leaving group L' may conveniently be a halogen atom, preferably a bromine or iodine atom, or a methanesulphonyl or <u>para</u>-toluenesulphonyl group.

Reaction of the compounds of formulae II and III may conveniently be effected in the presence of an inert solvent such as a $C_{1-3}$ alkanol e.g. methanol or ethanol. The base may then conveniently be for example an alkali metal $C_{1-3}$ alkoxide, which may readily be formed by dissolving the alkali metal, e.g. sodium, in the $C_{1-3}$ alkanol. Reaction may very conveniently be effected at reflux temperature.

The treatment with the strong acid, e.g. sulphuric acid, may also conveniently be effected at reflux temperature.

Reaction of a compound of formula I wherein X is oxygen and $R^2$ is hydrogen with an optionally substituted alkyl halide is conveniently achieved in the presence of a base such as an alkali metal hydride or amide, preferably lithium diisopropylamide, advantageously using a dipolar aprotic solvent such as tetrahydrofuran or dimethylsulphoxide. Reaction may conveniently be affected at ambient temperature.

Reaction of a compound of formula I wherein X is oxygen with a compound of formula $NH_2OR^{3'}$ may also conveniently be effected in the presence of an inert solvent such as a $C_{1-3}$alkanol, and at reflux temperature of the reaction mixture.

Reaction of a compound of formula I wherein $R^3(R^{3'})$ is hydrogen with a compound of formula $R^9L'$ is conveniently effected in the presence of an alkali metal hydroxide or carbonate or a trialkylamine base, advantageously in the presence of an inert solvent e.g. acetone, at reflux temperature.

Further in accordance with the invention there is provided a fungicidal composition which comprises a carrier and, as active ingredient, an imidazole of formula I or a salt thereof as defined above.

A composition according to the invention preferably contains from 0.5 to 95% by weight of active ingredient.

A carrier in a composition according to the invention is any material with which the active ingredient is formulated to facilitate application to the locus to be treated, which may for example be a plant, seed or soil, or to facilitate storage, transport or handling. A carrier may be a solid or a liquid, including a material which is normally gaseous but which has been compressed to form a liquid, and any of the carriers normally used in formulating fungicidal compositions may be used.

Suitable solid carriers include natural silicas such as diatomaceous earths; magnesium silicates, for example talcs; magnesium aluminum silicates, for example attapulgites and vermiculites; aluminium silicates, for example kaolinites, montmorillonites and micas; calcium carbonate; calcium sulphate; synthetic hydrated silicon oxides and synthetic calcium or aluminium silicates; elements, for example carbon and sulphur; natural and synthetic resins, for example coumarone resins, polyvinyl chloride, and styrene polymers and copolymers; solid polychlorophenols; bitumen; waxes, for example beeswax, paraffin wax, and chlorinated mineral waxes; and solid fertilisers, for example superphosphates.

Suitable liquid carriers include water; alcohols, for example isopropanol and glycols; ketones, for example acetone, methyl ethyl ketone, methyl isobutyl ketone and cyclohexanone; ethers; aromatic or araliphatic hydrocarbons, for example benzene, toluene and xylene; petroleum fractions, for example kerosine and light mineral oils; chlorinated hydrocarbons, for example carbon tetrachloride, perchloroethylene and trichloroethane. Mixtures of different liquids are often suitable.

Fungicidal compositions are often formulated and transported in a concentrated from which is subsequently diluted by the user before application. The presence of small amounts of a carrier which is a surface-active agent facilitates this process of dilution. Thus preferably at least one carrier

in a composition according to the invention is a surface-active agent. For example, a composition may contain at least two carriers, at least one of which is a surface-active agent.

Of particular interest in enhancing the duration of the protectant activity of the compounds of this invention is the use of a carrier which will provide a slow release of the fungicidal compounds into the environment of the plant which is to be protected. Such slow-release formulations could, for example, be inserted in the soil adjacent to the roots of a vine plant, or could include an adhesive component enabling them to be applied directly to the stem of a vine plant.

A surface-active agent may be an emulsifying agent, a dispersing agent or a wetting agent; it may be nonionic or ionic. Examples of suitable surface-active agents include the sodium or calcium salts of polyacrylic acids and lignin sulphonic acids; the condensation products of fatty acids or aliphatic amines or amides containing at least 12 carbon atoms in the molecule with ethylene oxide and/or propylene oxide; fatty acid esters of glycerol, sorbitan, sucrose or pentaerythritol; condensates of these with ethylene oxide and/or propylene oxide; condensation products of fatty alcohol or alkyl phenols, for example p-octylphenol or p-octylcresol, with ethylene oxide and/or propylene oxide; sulphates or sulphonates of these condensation products; alkali or alkaline earth metal salts, preferably sodium salts, of sulphuric or sulphonic acid esters containing at least 10 carbon atoms in the molecule, for example sodium lauryl sulphate, sodium secondary alkyl sulphates, sodium salts of sulphonated castor oil, and sodium alkylaryl sulphonates such as sodium dodecyl benzene sulponate; and polymers of ethylene oxide and copolymers of ethylene oxide and propylene oxide.

The compositions of the invention may for example be formulated as wettable powders, dusts, granules, solutions, emulsifiable concentrates, emulsions, suspension concentrates and aerosols. Wettable powders usually contain 25, 50 or 75%w of active ingredient and usually contain, in addition to solid inert carrier, 3-10%w of a dispersing agent and, where necessary, 0-10%w of stabiliser(s) and/or other additives such as penetrants or stickers. Dusts are usually formulated as a dust concentrate having a similar composition to that of a wettable powder but without a dispersant, and may be diluted in the field with further solid carrier to give a composition usually containing ½-10%w of active ingredient. Granules are usually prepared to have a size between 10 and 100 BS mesh (1.676-0.152mm), and may be manufactured by agglomeration or impregnation techniques.

Generally, granules will contain ½-25%w active ingredient and 0-10%w of additives such as stabilisers, slow release modifiers and binding agents. Emulsifiable concentrates usually contain, in addition to a solvent and, when necessary, co-solvent, 1-50%w/v active ingredient, 2-20%w/v emulsifiers and 0-20%w/v of other additives such as stabilisers, penetrants and corrosion inhibitors. Suspension concentrates are usually compounded so as to obtain a stable, non-sedimenting flowable product and usually contain 10-75%w of active ingredient, 0.5%-15%w of dispersing agents, 0.1-10%w of suspending agents such as protective colloids and thixotropic agents, 0-10%w of other additives such as defoamers, corrosion inhibitors, stabilisers, penetrants and stickers, and water or an organic liquid in which the active ingredient is substantially insoluble; certain organic solids or inorganic salts may be present dissolved in the formulation to assist in preventing sedimentation or as antifreeze agents for water.

The compositions may also contain other ingredients, for example other compounds possessing pesticidal, especially insecticidal, acaricidal, herbicidal or fungicidal, properties.

Aqueous dispersions and emulsions, for example compositions obtained by diluting a wettable powder or a concentrate according to the invention with water, also lie within the scope of the present invention. The said emulsions may be of the water-in-oil or of the oil-in-water type, and may have a thick 'mayonnaise'-like consistency.

The invention still further provides the use as a fungicide of a carboximidate of the general formula I as defined above, and a method for combating fungus at a locus, which comprises treating the locus, which may for example be plants subject to or subjected to fungal attack, seeds of such plants or the medium in which such plants are growing or are to be grown, with such a derivative.

The present invention is of wide applicability in the protection of crop plants against fungal attack. Typical crops which may be protected include vines, grain crops such as wheat and barley, rice, beans, tomatoes and apples. The duration of protection is normally dependent on the individual compound selected, and also a variety of external factors, such as climate, whose impact is normally mitigated by the use of a suitable formulation.

The invention will be further understood from the following examples.

EXAMPLE 1

Preparation of 5-[2-(4-chlorophenyl)-2-cyano-1-oxoethyl]-1-methylimidazole

Sodium (2.25g) was dissolved in methanol - (150ml). The resulting solution was heated under reflux and a solution of 1-methylimidazole-5-methyl carboxylate (9.0g) and p-chlorophenylacetonitrile - (10.8g) in methanol (50ml) was added dropwise thereto over 1 hour. The resulting mixture was heated under reflux for a further 4 hours and allowed to cool The methanol was evaporated off and the residue was diluted with water and washed with diethylether. Glacial acetic acid (7.6ml) was added to the aqueous phase and the resulting milky solution was extracted with chloroform (3 x 100ml). The combined chloroform extracts were washed with brine and dried (Mg $SO_4$), and solvent was evaporated off to yield the title compound as a solid (5.8g, 35%) mp 58-60°C.

Analysis requires: 60.2C; 3.9H; 16.2N

$C_{13}H_{10}ClN_3O$ found: 61.0C; 4.5H; 15.2N

EXAMPLE 2

Preparation of 5-[2-(4-chlorophenyl)-1-oxoethyl]-1-methylimidazole

5-[2-(4-Chlorophenyl)-2-cyano-1-oxoethyl]-1-methylimidazole (0.7g) was added portionwise to sulphuric acid (6ml, 13.5M) and heated under reflux for 5 hours and allowed to cool. The reaction mixture was poured into water (30ml) and the pH was adjusted to pH9 by addition of concentrated aqueous ammonia.

On cooling and scratching, the title compound precipitated out as a white solid (0.5g, 71%) mp 108-110°C.

Analysis requires: 61.1C; 4.6H; 11.7N

$C_{12}H_{11}ClN_2O$ found: 61.5C; 4.7H; 11.9N

EXAMPLE 3

Preparation of 5-[2(4-chlorophenyl)-1-oximinoethyl]-1-methylimidazole

5-[2-(4-Chlorophenyl)-1-oxoethyl]-1-methylimidazole (1g) was dissolved in industrial methylated spirits (20ml). To the resulting solution sodium acetate (0.35g) and hydroxylamine hydrochloride (0.3g) dissolved in water (5ml) were added and the reaction mixture was heated under reflux for 24 hours . On cooling, the solvent was evaporated off under reduced pressure and the residue was diluted with water and extracted with methylene chloride. The combined organic extracts were washed with water and dried ($MgSO_4$). The solvent was evaporated off to leave a solid which was washed with diethyl ether/petroleum ether - (1:1v/v) to afford the title product (0.75g, 70%) mp 172-3°C.

Analysis requires: 57.7C; 4.8H; 16.8N

$C_{13}H_{12}ClN_3O$ found: 57.4C; 5.0H; 16.2N

EXAMPLE 4

Preparation of 5-[2-(4-chlorophenyl)-1-methoxyiminoethyl]-1-methylimidazole

5-[2-(4-Chlorophenyl)-1-oxoethyl]-1-methylimidazole (1g) was dissolved in methanol - (20ml). Sodium acetate (0.72g) and methoxylamine hydrochloride (0.72g) in water (5ml) were added to the resulting solution and the reaction mixture was heated under reflux for 48 hours. On cooling, the solvent was evaporated off under reduced pressure and the residue was diluted with water and extracted with methylene chloride. The combined organic extracts were washed with water and dried - ($MgSO_4$) and the solvent was exaporated off under reduced pressure. The residual oil was chromatographed on silica using 3% v/v methanol/diethylether as eluant to afford the title product as an oil (0.4g, 35%).

Analysis requires: 59.2C; 5.3H; 15.9N

$C_{13}H_{14}ClN_3O$ found: 59.4C; 5.1H; 15.6N

EXAMPLE 5

Preparation of 5-[2-(4-chlorophenyl)-1-oxopropyl]-1-methylimidazole

To a stirred solution of lithium diisopropylamide [3.6ml of 1.4M solution of butyl lithium in hexane and diisopropylamine (0.5g)] at 0°C under nitrogen in dry tetrahydrofuran was added 5-[2-(4-chlorophenyl)-1-oxoethyl]-1-methylimidazole (1.0g) in dry tetrahydrofuran and stirring was continued for 1 hour. Methyl iodide (1g) in tetrahydrofuran - (10ml) was added dropwise and the reaction mixture was stirred at ambient temperature (20°C) for 12 hours. The reaction mixture was then diluted with water and extracted with methylene chloride. The combined organic extracts were washed with water and dried ($MgSO_4$), and the solvent compo-

nents were evaporated off under reduced pressure. A residual oil was obtained which was chromatographed on silica using 5% v/v methanol diethylether as eluant to afford the title product as an oil (10.9g, 79%).

Analysis requires: 62.9C; 5.2H; 11.3N

$C_{13}H_{13}ClN_2O$ found: 61.7C; 5.3H; 11.6N

EXAMPLE 6

Preparation of 5-[2-(4-chlorophenyl)-1-benzyloxyiminoethyl]-1-methylimidazole

5-[2-(4-Chlorophenyl)-1-oximinoethyl]-1-methylimidazole (1.0g) was heated under reflux with benzyl bromide (0.8g) and powdered potassium hydroxide (0.78g) in acetone (50ml) for 12 hours. On cooling, the solvent was evaporated off and the residue was diluted with water and extracted with chloroform. The combined organic extracts were washed with water and dried ($MgSO_4$) and the solvent was evaporated off under reduced pressure. The residual oil was chromatographed on alumina with diethyl ether as eluant to afford the title product as an oil (1.0g, 74%).

Analysis requires: 67.12C; 5.3H; 12.4N

$C_{19}H_{18}ClN_3O$ found: 68.3C; 5.7H; 10.5N

EXAMPLES 7 to 20

By processes analogous to those of Examples 1 to 6 there were prepared a number of other compounds of the invention, as detailed in Table I following:

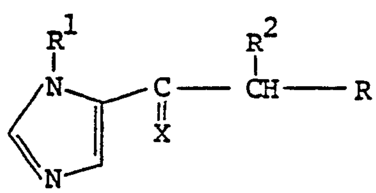

TABLE I

| Example | $R^1$ | $R^2$ | R | X | mp (°C) | Analysis Found (Theory) |
|---|---|---|---|---|---|---|
| 7 | $CH_3$ | H | 4-chlorophenyl | $NOC_2H_5$ | oil | 59.6C; 6.0H; 14.4N (60.5C; 5.8H; 15.1N) |
| 8 | HCl Salt of Example 7 compound | | | | 222-223°C | 52.4C; 5.5H; 13.5N (53.5C; 5.5H; 14.5N) |
| 9 | $CH_3$ | H | 4-chlorophenyl | $NOCH_2CH=CH_2$ | oil | 61.7C; 5.7H; 14.3N (62.2C; 5.5H; 14.5N) |
| 10 | HCl Salt of Example 9 compound | | | | 220-221°C | 55.1C; 5.2H; 12.9N (55.2C; 5.2H; 12.9N) |
| 11 | $CH_3$ | CN | 2,4-dichlorophenyl | O | 120-122°C | 52.5C; 3.1H; 13.7N (53.2C; 3.1H; 14.3N) |
| 12 | $CH_3$ | H | 2,4-dichlorophenyl | O | 128-130°C | 53.9C; 3.3H; 9.7N (53.5C; 3.7H; 10.4N) |
| 13 | $CH_3$ | H | 2,4-dichlorophenyl | $NOCH_3$ | oil | 51.1C; 4.4H; 13.7N (52.3C; 4.4H; 14.1N) |

Table I (continued)

| Example | $R^1$ | $R^2$ | R | X | mp(°C) | Analysis Found (Theory) |
|---|---|---|---|---|---|---|
| 14 | HCl Salt of Example 13 compound | | | | 207-208°C | 44.1C; 4.3H; 11.9N<br>(46.6C; 4.2H; 12.5N) |
| 15 | $CH(CH_3)_2$ | CN | 4-chlorophenyl | O | 193-195°C | |
| 16 | $CH(CH_3)_2$ | H | 4-chlorophenyl | O | 57-58°C | 63.3C; 5.7H; 10.6N<br>(64.0C; 5.7H; 10.7N |
| 17 | $CH(CH_3)_2$ | H | 4-chlorophenyl | NOH | 166-167°C | 59.6C; 5.7H; 15.1N<br>(60.5C; 5.8H; 15.1N) |
| 18 | $CH(CH_3)_2$ | H | 4-chlorophenyl | $NOCH_3$ | oil | 59.4C; 6.1H; 13.0N<br>(61.8C; 6.2H; 14.4N) |
| 19 | $CH_3$ | H | 2,4-dichlorophenyl | $NOCH_2CH=CH_2$ | oil | 55.2C; 5.2H; 12.7N<br>(55.6C; 4.6H; 13.0N) |
| 20 | $CH_3$ | H | 2,4-dichlorophenyl | $NOC_2H_5$ | 52-53°C | 53.8C; 4.9H; 13.1N<br>(53.8C; 4.8H; 13.5N) |

Table I (continued)

| Example | $R^1$ | $R^2$ | R | X | mp(°C) | Analysis Found (Theory) |
|---|---|---|---|---|---|---|
| 21 | $CH_3$ | $CH_3$ | 2,4-dichlorophenyl | O | oil | 61.7C; 11.6H; 5.3N<br>(62.9C; 11.3H; 5.2N) |
| 22 | $CH_3$ | $CONH_2$ | 2,4-dichlorophenyl | O | 191-192°C | 46.2C; 3.6H; 12.7N<br>(50.0C; 3.5H; 13.5N) |
| 23 | $CH_3$ | H | 4-chlorophenyl | NO-2,4-di-chlorobenzyl | oil | 55.8C; 4.1H; 8.7N<br>(55.8C; 3.9H; 10.3N) |
| 24 | $CH_3$ | H | 2,4-dichlorophenyl | NOH | 227-229°C | 51.0C; 4.2H; 14.7N<br>(50.7C; 3.9H; 14.8N) |

EXAMPLE 21

The fungicidal activity of compounds of the invention was investigated by means of the following tests.

(a) Direct protectant activity against vine downy mildew (Plasmopara viticola; Pvp)

The test is a direct protectant one, using a foliar spray. The lower surfaces of leaves of whole vine plants (cv Cabernet Sauvignon) are sprayed with a solution of active material in 1:1v/v water/acetone containing 0.04%w "Triton X-155" - (trade mark) (octylphenol polyoxyethylene surfactant), at a dosage of 1 kilogram of active material per hectare using a track sprayer which delivers 620 l/ha, and after a subsequent 24 hours under normal glasshouse conditions the lower surfaces of the leaves are inoculated by spraying with an aqueous solution containing $10^4$ zoosporangia/ml. The inoculated plants are kept for 24 hours in a high humidity compartment, 5 days under normal glasshouse conditions and then returned for a further 24 hours to high humidity. Assessment is based on the percentage of leaf area covered by sporulation compared with that on control leaves.

(b) Direct protectant activity against vine grey mould (Botrytis cinerea; Bcp)

The test is a direct protectant one using a foliar spray and is effected as described under (a), with the difference that the leaves are inoculated by spraying with an aqueous solution containing $10^5$ conidia/ml.

(c) Activity against wheat leafspot (Leptosphaeria nodorum; Ln.)

The test is a direct antisporulant one, using a foliar spray. Leaves of wheat plants (cv Mardler), at the single leaf stage, are inoculated by spraying with an aqueous suspension containing $8 \times 10^5$ spores/ml. The inoculated plants are kept for 24 hours in a high humidity compartment prior to treatment. The plants are sprayed at a dosage of 1 kg. of active material per hectare using a track sprayer as described under (a). After drying, the plants are kept for 5 days under normal glasshouse conditions, followed by assessment. Assessment is based on the percentage of leaf area covered by sporulation compared with that on leaves of control plants.

(d) Activity against barley powdery mildew : (Erysiphe graminis f.sp. hordei; Eg)

The test is a direct antisporulant one, using a foliar spray. Leaves of barley seedlings, cultivar Golden Promise, are inoculated by dusting with mildew conidia one day prior to treatment with the test compound. The inoculated plants are kept overnight at glasshouse ambient temperature and humidity prior to treatment. The plants are sprayed at a dosage of 1kg. of active material per hectare using a track sprayer as described under (a). After drying, plants are returned to a compartment at ambient temperature and humidity for up to 7 days, followed by assessment. Assessment is based on the percentage of leaf area covered by sporulation compared with that on leaves of control plants.

(e) Activity against apple powdery mildew : (Podosphaera leucotricha; Pl)

The test is a direct anti-sporulant one using a foliar spray. The upper surfaces of leaves of whole apply seedlings are inoculated by spraying with an aqueous suspension containing $10^5$ conidia/ml 2 days prior to treatment with the test compound. The inoculated plants are immediately dried and kept at glasshouse ambient temperatures and humidity prior to treatment. The plants are sprayed at a dosage of 1 kilogram of active material per hectare using a track sprayer as described under (a). After drying the plants are returned to a compartment at ambient temperature and humidity for up to 9 days, followed by assessment. Assessment is based on the percentage of the leaf area covered by sporulation compared with that on leaves of control plants.

(f) Activity against broad bean rust (Uromyces fabae Uf)

The test is a direct antisporulant one using foliar spray. Pots containing 1 plant per pot were inoculated by spraying an aqueous suspension, containing $5 \times 10^4$ spores/ml plus a little "Triton X-155", onto the upper surface of each leaf 20-24 hours before treatment with test compound. The inoculated plants were kept overnight in a high humidity compartment, dried at glass-house ambient temperature and then sprayed, on the leaf upper surface, at a dosage of 1kg/ha of active

material using a track sprayer as described under (a). After treatment the plants were kept at glasshouse temperature and assessment made 11-14 days after treatment. Symptoms are assessed on the relative density of sporulating pustules per plant compared with that on control plants.

(g) Activity against rice leaf blast (*Pyricularia oryzae* Po)

The test is a direct eradicant one using a foliar spray. The leaves of rice seedlings (about 30 seedlings per pot) are sprayed with an aqueous suspension containing $10^5$ spores/ml 20-24 hours prior to treatment with the test compound. The inoculated plants are kept overnight in high humidity and then allowed to dry before spraying at a dosage of 1kg of active material per hectare using a track sprayer as described under (a). After treatment the plants are kept in a rice compartment at 25-30°C and high humidity. Assessments are made 4-5 days after treatment and are based on the density of necrotic lesions and the degree of withering when compared with control plants.

(h) Activity against tomato early blight (*Alternaria solani* As)

The test is a direct protectant one using a foliar spray. The upper surfaces of leaves of young tomato plants are sprayed with a solution of active material as described in (a) above. After 24hrs under normal glasshouse conditions, the upper surgaces of the leaves are inoculated by spraying with an aqueous suspension containing $10^4$ spores/ml. The inoculated plants are kept for 72 hours in a high humidity compartment and are then removed to lower humidity (50-70% relative humidity). Assessment is made 8 days after inoculation.

The extent of disease control in all the above tests is expressed as a rating compared with a diluent sprayed control according to the criteria:-

0 = less than 50% disease control

1 = about 50-80% disease control

2 = greater than 80% disease control.

(i) Activity against wheat eyespot (*Pseudocereosporella herpotrichoides* Ph)

The test is an in vitro one. Samples are prepared wherein 0.7ml solution containing 2mg active material dissolved in acetone is evenly dispersed in 20ml molten half-strength potato dextrose agar - (formed by dissolving 2g potato extract, 10g dextrose and 7.5g agar in 1 litre of water and sterilising for 15 minutes at 121°C) and the resulting 20ml portions are allowed to set in 9cm petri dishes. The concentration of active material in the resulting samples is 100ppm. Upon settling, two plugs of 5mm diameter taken from the advancing edge of a stock plate of 3 to 4 week old culture of *P. herpotrichoides* on full strength potato dextrose agar, incubated at 20-22°C in darkness, are placed, equally spaced on the surface of each sample, mycelial side uppermost.

The samples are incubated for 11 days at 20-22°C in darkness before assessment. Diametric growth is measured with the width of the plug subtracted and results compared with growth on a sample wherein 0.7ml acetone containing no active material is dispersed in 20ml half-strength potato agar. Treatments are graded:-

0 = less than 50% disease control

1 = about 50-80% disease control

2 = greater than 80% disease control

Results of the above tests are given in Table II following:

TABLE II

| Compound Example | Fungicidal Activity | | | | | | | | |
|---|---|---|---|---|---|---|---|---|---|
| | Pvp | Bcp | Ln | Eg | Pl | Uf | Po | As | Ph |
| 1 | | 1 | | | | | | | |
| 2 | | | | | | | | 2 | |
| 3 | | | | 2 | 1 | | | 2 | 2 |
| 4 | 2 | 2 | | 2 | | | | 2 | 2 |
| 5 | | 2 | 2 | 2 | | | | | 2 |
| 6 | 2 | | | 2 | 1 | | 1 | | 2 |
| 7 | | | | 2 | 2 | | | | 2 |
| 8 | | 1 | 2 | 2 | 2 | | | | 2 |
| 9 | | 1 | | 2 | 2 | | | | 2 |
| 10 | | | | 1 | | | | | 2 |
| 11 | | | 1 | 2 | | | | 1 | |
| 12 | | 1 | | 2 | | | 1 | | |
| 13 | | 2 | 2 | 2 | 2 | 2 | | | 2 |
| 14 | | 1 | 1 | 2 | 2 | 2 | | | 2 |
| 16 | | | | 2 | 2 | | 2 | | 2 |
| 17 | 1 | | | 2 | | | | | |
| 18 | | 1 | | 2 | 2 | | | | 2 |
| 19 | 1 | | | 2 | | | | | 2 |
| 20 | 2 | | 2 | 2 | 2 | 2 | | | 2 |
| 21 | | | 1 | 2 | 2 | | | 2 | 2 |
| 22 | | | | 2 | | | | | |
| 23 | | | | 2 | | | | | |
| 24 | 1 | | | 2 | 2 | | | | |

**Claims**

1. An imidazole of general formula

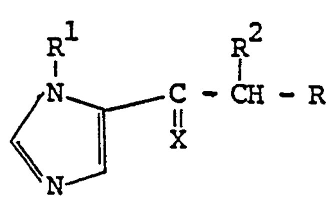

(I)

or an acid-addition salt thereof, wherein R represents an optionally substituted phenyl group, $R^1$ represents an optionally substituted alkyl, cycloalkyl, alkenyl, aryl or aralkyl group, $R^2$ represents a hydrogen atom, a cyano group, a $-CONH_2$ group, or an optionally substituted alkyl group, and X represents an oxygen atom or a =NOR³ moiety where $R^3$ is a hydrogen atom or an optionally substituted alkyl, cycloalkyl, alkenyl, alkylcarbonyl, aryl or aralkyl group.

2. An imidazole according to Claim 1 wherein R is a phenyl group optionally substituted by one to three halogen atoms.

3. An imidazole according to Claim 2 wherein the halogen atoms are chlorine atoms.

4. An imidazole according to any of Claims 1 to 3 wherein $R^1$ is a $C_{1-10}$ alkyl group, a $C_{3-8}$ cycloalkyl group, a $C_{2-6}$alkenyl group, a phenyl group or a benzyl group.

5. An imidazole group according to Claim 4 wherein $R^1$ is a $C_{1-6}$ alkyl group.

6. An imidazole according to any of Claims 1 to 5 wherein $R^2$ represents a hydrogen atom or a $C_{1-6}$ alkyl group.

7. An imidazole according to Claim 6 wherein $R^2$ represents a hydrogen atom.

8. An imidazole according to any of Claims 1 to 7 wherein X represents a =$NOR^3$ moiety wherein $R^3$ is a hydrogen atom, a $C_{1-6}$ alkyl group, a $C_{3-8}$ cycloalkyl group, a $C_{2-6}$ alkenyl group, a ($C_{1-6}$ alkyl)carbonyl group, a phenyl group or a benzyl group optionally substituted by one to three substituents selected from halogen atoms and methyl, methoxy, cyano and trifluoromethyl groups.

9. An imidazole according to Claim 8 wherein $R^3$ is a hydrogen atom, a C $_{1-6}$ alkyl group or a $C_{3-6}$ alkenyl group wherein the double bond is not adjacent the oxygen atom of the =$NOR^3$ moiety.

10. An imidazole according to Claim 1 substantially as hereinbefore described in any one of Examples 1 to 18.

11. A process for the preparation of an imidazole of formula I as defined in any of Claims 1 to 8 which comprises reacting an imidazole ester of formula

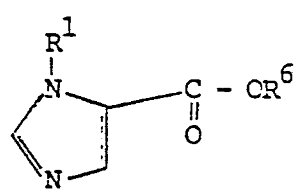

(II)

where $R^1$ is as defined in Claim 1 and $R^6$ is an alkyl group with a compound of formula

$R^7$-$CH_2$-R (III)

wherein R is as defined in Claim 1 and $R^7$ is -CN or -$COOR^8$ where $R^8$ is an alkyl group, in the presence of a base, followed, optionally when $R^7$ is -CN, by treatment with a strong acid to produce a compound of formula I wherein X is oxygen and $R^2$ is hydrogen or -$CONH_2$, optionally followed by reaction with an optionally substituted alkyl halide in the presence of a base to produce a compound of formula I wherein X is oxygen and $R^2$ is an optionally substituted alkyl group, optionally followed by reaction with a compound of formula $NH_2OR^{3'}$, where $R^{3'}$ is a hydrogen atom or an optionally substituted alkyl, cycloalkyl, alkenyl, aryl or aralkyl group, and optionally followed, in the case where $R^{3'}$ is hydrogen, by reaction with an acid derivative $R^4COL$, where $R^4$ is alkyl and L is a leaving group, or with a compound of formula $R^5L'$, where $R^5$ is an optionally substituted alkyl, cycloalkyl, alkenyl or aralkyl group and L' is a leaving group.

12. A process according to Claim 11 substantially as hereinbefore described with reference to any of Examples 1 to 18.

13. An imidazole of formula I whenever prepared by a process according to Claim 11 or 12.

14. A fungicidal composition which comprises a carrier and, as active ingredient, an imidazole according to any of Claims 1 to 10 or Claim 13.

15. A composition according to Claim 14, which comprises at least two carriers, at least one of which is a surface-active agent.

16. A method of combating fungus at a locus, which comprises treating the locus with an imidazole of the general formula I as defined in any one of Claims 1 to 10 or Claim 13.

17. A method as claimed in Claim 16, in which the locus comprises plants subject or subjected to fungal attack, seeds of such plants or the medium in which the plants are growing or are to be grown.

18. The use as a fungicide of an imidazole of the general formula I as defined in any one of Claims 1 to 10 or Claim 13.

European Patent Office

# EUROPEAN SEARCH REPORT

Application number

EP 86201606.0

| Category | Citation of document with indication, where appropriate, of relevant passages | Relevant to claim | CLASSIFICATION OF THE APPLICATION (Int. Cl.4) |
|---|---|---|---|
| A | DD - A - 156 260 (FARMOS GROUP LTD.)<br>* Claim 1 *<br>-- | 1-3,11 | C 07 D 233/64<br>A 01 N 43/50 |
| D,A | EP - A1 - 0 058 047 (FARMOS-YHTYMA OY)<br>* Claim 1; page 8, lines 11,12 *<br>---- | 1-3,11,<br>14,18 | |

TECHNICAL FIELDS SEARCHED (Int. Cl.4)

C 07 D 233/00
A 01 N 43/00

The present search report has been drawn up for all claims

| Place of search | Date of completion of the search | Examiner |
|---|---|---|
| VIENNA | 22-12-1986 | BRUS |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document